(19)

Europäisches Patentamt
European Patent Office
Office européen des brevets

(11) **EP 4 209 774 A1**

(12) **EUROPEAN PATENT APPLICATION**
published in accordance with Art. 153(4) EPC

(43) Date of publication:
**12.07.2023 Bulletin 2023/28**

(21) Application number: **22873960.3**

(22) Date of filing: **22.08.2022**

(51) International Patent Classification (IPC):
$G01N\ 3/00\ ^{(2006.01)}$    $G01N\ 3/06\ ^{(2006.01)}$
$G01N\ 3/08\ ^{(2006.01)}$    $G01N\ 3/24\ ^{(2006.01)}$
$G01N\ 3/60\ ^{(2006.01)}$    $G01N\ 33/44\ ^{(2006.01)}$

(52) Cooperative Patent Classification (CPC):
G01N 3/00; G01N 3/06; G01N 3/08; G01N 3/24;
G01N 3/60; G01N 33/44

(86) International application number:
**PCT/KR2022/012503**

(87) International publication number:
**WO 2023/068520 (27.04.2023 Gazette 2023/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.10.2021 KR 20210138914
19.10.2021 KR 20210138918
13.07.2022 KR 20220086160**

(71) Applicant: **LG CHEM, LTD.
Yeongdeungpo-gu,
Seoul 07336 (KR)**

(72) Inventors:
• **IM, Dam Hyeok**
  **Yuseong-gu, Daejeon 34122 (KR)**
• **MOON, Sung Nam**
  **Yuseong-gu, Daejeon 34122 (KR)**
• **JUNG, Jin Mi**
  **Yuseong-gu, Daejeon 34122 (KR)**

(74) Representative: **Goddar, Heinz J.
Boehmert & Boehmert
Anwaltspartnerschaft mbB
Pettenkoferstrasse 22
80336 München (DE)**

(54) **DEVICE AND METHOD FOR PREDICTING PHYSICAL PROPERTIES OF MULTILAYER MATERIAL**

(57) A system and method for predicting physical properties such as elastic moduli, shear moduli, and Poisson's ratios (Poisson's ratio) of an entire laminate are provided. The system and method can determine not only homogenized stiffness of the entire laminate of the multilayer material but also physical property values or a thickness of an undetermined layer, when developing a multilayer material having a laminated structure.

【FIG. 1】

EP 4 209 774 A1

**Description**

[Technical Field]

This application claims priority to and the benefit of Korean Patent Application No. 10-2021-0138914, filed on October 19, 2021, Korean Patent Application No. 10-2021-0138918, filed on October 19, 2021, and Korean Patent Application No. 10-2022-0086160, filed on July 13, 2022, the disclosures of which are incorporated herein by reference in their entireties.

The present invention relates to a system and method for predicting the physical properties of a multilayer material.

[Background Art]

A polymer film refers to a non-fibrous flat plastic molded article, which is light, has a property as a good barrier, has high transparency, and is relatively inexpensive, so it is used in many fields such as packaging materials, household goods, electronic devices, automobiles, and aircraft.

For example, synthetic polymers such as polyethylene (PE), polypropylene (PP), polyvinyl chloride (PVC), and polyethylene terephthalate (PET) are processed into a polymer film and widely used in Korea and abroad, and currently, many synthetic polymers are used as materials for polymer films either alone or by blending.

A multilayer film is a composite film in which different types of films are laminated for the purpose of multi-functionality of the film, and various types of multilayer films formed of, for example, the combination of a film having the excellent mechanical properties of polyethylene (PE) and the printing aesthetics of cellophane, and nylon and a vinyl alcohol-ethylene copolymer are used as packaging materials.

In the case of developing such a multilayer film as a material, it is necessary to predict physical properties such as the elastic moduli, the shear moduli, and the Poisson's ratios of the entire laminate.

When the elastic modulus is referred to as "E," the shear modulus is referred to as "G," and the Poisson's ratio is referred to as "$\upsilon$," the relationship between them is as follows:

$$G = \frac{E}{2(1+\nu)}$$

The material has a property in which lateral transformation (transformation in the direction proportional to a load) and longitudinal transformation (transformation in the direction of load) are proportional to each other within the elastic range, and the ratio of these two transformations have a certain value according to a material within the elastic limit and is called the Poisson's ratio.

In addition, when the elastic modulus represents the degree of strains occurring when an elastic material is subjected to stress, and the elastic modulus is referred to as "E," the stress is referred to as "$\sigma$," and the strain is referred to as "$\varepsilon$," the relationship between them is as follows.

$$E = \frac{\sigma}{\varepsilon}$$

Meanwhile, since materials for a multilayer film are anisotropic in a machine direction (MD) and a transverse direction (TD) during the manufacturing process, for the robust design of materials, it is necessary to receive not only the homogenized stiffness of an entire laminate of a multilayer film but also only one of a physical property value and a thickness of one undetermined film as range values, and extract the other values through an algorithm.

For example, in Korean Unexamined Patent Application Publication No. 2005-0112788 (published on December 01, 2005), titled "Evaluation of effective stiffness of composite beam and method of optimizing lamination angle thereof," a method of evaluating effective stiffness of a composite beam made by laminating plies consisting of a novel material, which includes (a) calculating the stiffness of each of the plies; (b) calculating the stiffness matrix of the entire laminated composite beam; and (c) deriving an effective stiffness value of the composite beam by applying the stiffness matrix and a strain to a resulting stress component formula, has been disclosed.

However, the technology disclosed in Korean Unexamined Patent Application Publication No. 2005-0112788 does not reset the stiffness matrix, so there is a problem that it is not suitable for the application to the development of a material for a multilayer film like in the present invention.

[Disclosure]

[Technical Problem]

To solve technical problems of the present invention, the present invention is directed to providing a system and method for predicting the physical properties of a multilayer material, which can predict physical properties such as the elastic moduli, shear moduli, and the Poisson's ratios of the entire laminate, and can extract not only a homogenized stiffness of the entire laminate of the multilayer material but also a physical property value or thickness of an undetermined layer, when a multilayer material having a laminate structure is developed.

[Technical Solution]

As an aspect for achieving the above purposes, the present invention provides a system for predicting the physical properties of a multilayer material.

In one embodiment, the system of the present invention includes an input unit for inputting (a) any one or more of physical properties such as an elastic modulus, Poisson's ratio, shear modulus, thickness, lamination angle, or a set number of zones of each layer, except for an undetermined layer, and (b) any one or more physical properties of an elastic modulus or a thickness of the undetermined layer;

> a control unit which is connected to the input unit and includes an entire laminate property calculation unit and an undetermined layer property calculation unit;
> a display that is connected to the control unit; and
> a storage unit that is connected to the control unit.

In another aspect for achieving the above purposes, the present invention provides a method of predicting the physical properties of a multilayer material.

In one embodiment, the method of predicting the physical properties of a multilayer material in which two or more materials are laminated according to the present invention includes

> inputting any one or more of physical properties of an elastic modulus, a Poisson's ratio, a shear modulus, a thickness, a lamination angle, or a set number of zones of each layer, except for an undetermined layer, and any one or more physical properties of an elastic modulus or a thickness of the undetermined layer as input values;
> calculating the physical properties of the multilayer material, which is an entire laminate, using the input physical properties; and
> determining whether the calculated physical properties converge to final target physical properties.

[Advantageous Effects]

The present invention can predict, when a multilayer material having a laminate structure is developed, physical properties such as the elastic moduli, shear moduli, and Poisson's ratios of an entire laminate, and can extract not only a homogenized stiffness of the entire laminate of the multilayer material but also a physical property value or thickness of an undetermined layer.

[Brief Description of the Drawings]

FIG. 1 is a diagram illustrating a configuration of a system for predicting the physical properties of a multilayer material according to a first embodiment of the present invention.
FIG. 2 is a flowchart of a method of predicting the physical properties of a multilayer material according to a second embodiment of the present invention.
FIG. 3 is a flowchart of a method of predicting the physical properties of a multilayer material according to a third embodiment of the present invention.
FIG. 4 is a graph showing the correlation between elastic moduli ($E_{1,2}$) and a thickness (z) of an undetermined layer according to the present invention.

[Detailed Description of the Preferred Embodiments]

As an aspect for achieving the above purposes, the present invention provides a system for predicting the physical properties of a multilayer material.

In one embodiment, the system of the present invention includes an input unit for inputting (a) any one or more physical properties of an elastic modulus, a Poisson's ratio, a shear modulus, a thickness, a lamination angle, or a set number of zones of each layer, except for an undetermined layer, and (b) any one or more physical properties of an elastic modulus or a thickness of the undetermined layer;

a control unit which is connected to the input unit and includes an entire laminate property calculation unit and an undetermined layer property calculation unit;
a display that is connected to the control unit; and
a storage unit that is connected to the control unit.

In an exemplary embodiment, the control unit sets compliance matrices for stiffness matrices of the multilayer material, which is an entire laminate, using the input physical properties, calculates physical properties of the multilayer material, which is the entire laminate, using values of the set compliance matrices, and determines whether the calculated physical properties converge to the final target physical properties.

In one exemplary embodiment,

a system of the present invention includes an input unit for inputting (a) an elastic modulus, a Poisson's ratio, a shear modulus, a thickness, a lamination angle, and a set number of zones of each layer, except for an undetermined layer, and (b) any one or more of an elastic modulus or thickness of the undetermined layer;
a control unit which is connected to the input unit and includes an entire laminate property calculation unit and an undetermined layer property calculation unit;
a display that is connected to the control unit; and
a storage unit that is connected to the control unit.

In another exemplary embodiment, the control unit, for the multilayer material in which two or more materials are laminated, receives (a) the elastic modulus, Poisson's ratio, shear modulus, thickness, lamination angle, and a set number of zones of each layer, except for an undetermined layer, and (b) any one or more of the elastic modulus or thickness of the undetermined layer.

Here, the control unit calculates a stiffness matrix of each layer using the elastic modulus, Poisson's ratio, and shear modulus of each layer, except for the undetermined layer, resets the stiffness matrix of each layer by reflecting the lamination angle of each layer in the obtained stiffness matrix, calculates stiffness matrices of the multilayer material, which is the entire laminate, using a value of the stiffness matrix reset by receiving the thickness information of each layer, sets compliance matrices for the calculated stiffness matrices of the multilayer material, which is the entire laminate, and calculates any one or more of elastic moduli ($/E_{x,y}$), Poisson's ratios ($/\upsilon_{x,y}$), or shear moduli ($/G_{x,y}$) of the multilayer material, which is the entire laminate, using the total thickness and values of the set compliance matrices of the multilayer material, which is the entire laminate, and
determines whether any one or more of the calculated elastic moduli ($/E_{x,y}$), Poisson's ratios ($/\upsilon_{x,y}$) and shear moduli ($/G_{x,y}$) of the multilayer material converge to any one or more of a target elastic moduli (target $/E_{x,y}$), target Poisson's ratios (target $/\upsilon_{x,y}$) or a target shear moduli (target $/G_{x,y}$) received as the final target physical properties.

In the present invention, only one of the physical property value and thickness of the undetermined layer is input as a range value, and the other value may be extracted through an algorithm. In another example, the present invention can simultaneously input the physical property value and thickness of the undetermined layer as range values, and extract or verify a value within these ranges. Furthermore, when there is no value satisfying the physical property value and thickness ranges of the undetermined layer, it is possible to fix each range value, extract the other value, and present the same to a user.

In one embodiment,
in the case in which the elastic modulus is selected from the elastic modulus and thickness of the undetermined layer and input, when any one or more of the calculated elastic moduli ($/E_{x,y}$), Poisson's ratios ($/\upsilon_{x,y}$), or shear moduli ($/G_{x,y}$) of the multilayer material do not converge to any one or more of the target elastic moduli (target $/E_{x,y}$), target Poisson's ratios (target $/\upsilon_{x,y}$), or target shear moduli (target $/G_{x,y}$) received as the final target physical properties, the control unit may update the elastic modulus value of the undetermined layer.

In another embodiment,
in a case in which the thickness is selected from the elastic modulus and thickness of the undetermined layer and input, when any one or more of the calculated elastic moduli ($/E_{x,y}$), Poisson's ratios ($/\upsilon_{x,y}$), or shear moduli ($/G_{x,y}$) of the multilayer material do not converge to any one or more of the target elastic moduli (target $/E_{x,y}$), target Poisson's ratios (target $/\upsilon_{x,y}$), or target shear moduli (target $/G_{x,y}$) received as the final target physical properties, the control unit may update a value of the thickness of the undetermined layer.

In still another embodiment, the control unit may output any one or more of the elastic modulus, Poisson's ratio, shear modulus, or thickness of the undetermined layer when any one or more of the calculated elastic moduli ($/E_{x,y}$), Poisson's ratios ($/\upsilon_{x,y}$), or shear moduli ($/G_{x,y}$) of the multilayer material converge to any one or more of the target elastic moduli (target $/E_{x,y}$), target Poisson's ratios (target $/\upsilon_{x,y}$), or target shear moduli (target $/G_{x,y}$) received as the final target physical properties.

In yet another embodiment, the control unit may plot the correlation between the elastic modulus and thickness of the undetermined layer in a graph.

As another aspect to achieve the above purposes, the present invention provides a method of predicting the physical properties of a multilayer material.

In one embodiment,

the method of predicting the physical properties of a multilayer material in which two or more materials are laminated according to the present invention includes

inputting any one or more of physical properties of an elastic modulus, a Poisson's ratio, a shear modulus, a thickness, a lamination angle, or a set number of zones of each layer, except for an undetermined layer, and any one or more physical properties of an elastic modulus or a thickness of the undetermined layer as input values;

calculating physical properties of the multilayer material, which is an entire laminate, using the input physical properties; and

determining whether the calculated physical properties converge to the final target physical properties.

In one exemplary embodiment, the method of predicting the physical properties of a multilayer material according to the present invention includes

inputting any one or more of an elastic modulus, Poisson's ratio, shear modulus, thickness, lamination angle, or set number of zones of each layer, except for an undetermined layer;

inputting any one or more physical properties of an elastic modulus or thickness of the undetermined layer;

calculating physical properties of the multilayer material, which is an entire laminate, using the input physical properties; and

determining whether the calculated physical properties converge to the final target physical properties.

In an exemplary embodiment, the calculating of physical properties of a multilayer material includes

setting compliance matrices for stiffness matrices of the multilayer material, which is the entire laminate; and

calculating physical properties of the multilayer material, which is the entire laminate, using values of the set compliance matrices.

In one embodiment, the inputting as input values includes

inputting an elastic modulus, a Poisson's ratio, a shear modulus, a thickness, a lamination angle and a set number of zones of each layer, except for an undetermined layer (first step); and

inputting any one or more of an elastic modulus or thickness of the undetermined layer (second step).

In another embodiment, the calculating of physical properties of the multilayer material includes

calculating a stiffness matrix of each layer, except for an undetermined layer, using the elastic modulus, Poisson's ratio, and shear modulus (third step);

resetting a stiffness matrix of each layer by reflecting the lamination angle of each layer in the obtained stiffness matrix (fourth step);

calculating stiffness matrices of the multilayer material, which is the entire laminate, using a value of the stiffness matrix reset by receiving the thickness information of each layer (fifth step);

setting compliance matrices for the calculated stiffness matrices of the multilayer material, which is the entire laminate (sixth step); and

calculating elastic moduli, Poisson's ratios, and shear moduli of the multilayer material, which is the entire laminate, using the total thickness and values of the set compliance matrices of the multilayer material, which is the entire laminate (seventh step).

In still another embodiment, the determining whether the calculated properties converge to the final target physical properties includes

inputting an elastic modulus, a Poisson's ratio, and a shear modulus as the final target physical properties (eighth step); and
determining whether the calculated elastic moduli ($/E_{x,y}$), Poisson's ratios ($/\upsilon_{x,y}$), and shear moduli ($/G_{x,y}$) converge to the target elastic moduli (target $/E_{x,y}$), target Poisson's ratios (target $/\upsilon_{x,y}$), and target shear moduli (target $/G_{x,y}$) received as the final target physical properties (ninth step).

In an exemplary embodiment, in the third step, stiffness matrices ($[Q]^k_{1,2}$) in the machine direction (1) and the transverse direction (2) of each layer (k) can be calculated using elastic moduli ($E^k_{1,2}$), Poisson's ratios ($\upsilon^k_{1,2}$), and shear moduli ($G^k_{1,2}$) as shown in Equation 1 below.

$$[\text{Equation 1}]$$

$$\begin{bmatrix} \sigma_1 \\ \sigma_2 \\ \tau_6 \end{bmatrix} = \begin{bmatrix} Q_{11} & Q_{12} & 0 \\ Q_{12} & Q_{22} & 0 \\ 0 & 0 & Q_{66} \end{bmatrix} \begin{bmatrix} \varepsilon_1 \\ \varepsilon_2 \\ \gamma_6 \end{bmatrix}$$

$$Q_{11} = \frac{E_1}{1 - V_{12}V_{21}} \qquad Q_{22} = \frac{E_2}{1 - V_{12}V_{21}}$$

$$Q_{12} = Q_{21} = \frac{V_{21}E_1}{1 - V_{12}V_{21}} = \frac{V_{12}E_2}{1 - V_{12}V_{21}}$$

$$Q_{66} = G_{12}$$

In Equation 1, for an isotropic material, G = E / 2(1+$\upsilon$).

In an exemplary embodiment, in the fourth step, stiffness matrices ($[Q]^k_{x,y}$) of each layer (k) can be reset as shown in Equation 2 below by reflecting the lamination angle ($\theta^k$) of each layer (k) in the obtained stiffness matrices ($[Q]^k_{1,2}$).

$$[\text{Equation 2}]$$

$$[T] = \begin{bmatrix} m^2 & n^2 & 2mn \\ n^2 & m^2 & -2mn \\ -mn & mn & m^2 - n^2 \end{bmatrix} \quad m=\cos\theta, \ n=\sin\theta$$

$$\begin{bmatrix} Q_{xx} & Q_{xy} & 2Q_{xs} \\ Q_{xy} & Q_{yy} & 2Q_{ys} \\ Q_{xs} & Q_{ys} & 2Q_{ss} \end{bmatrix} = [T^{-1}] \begin{bmatrix} Q_{11} & Q_{12} & 0 \\ Q_{12} & Q_{22} & 0 \\ 0 & 0 & 2Q_{66} \end{bmatrix} [T]$$

$$\begin{bmatrix} \sigma_x \\ \sigma_y \\ \tau_s \end{bmatrix} = \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix} \begin{bmatrix} \varepsilon_x \\ \varepsilon_y \\ \gamma_s \end{bmatrix} \quad \begin{array}{l} \text{Stress-strain relation reflecting angle} \\ (\text{dir-x,y}) \end{array}$$

In an exemplary embodiment, according to the method of the present invention, in the fifth step, it is possible to calculate stiffness matrices ($[A]_{x,y}$, $[B]_{x,y}$, $[D]_{x,y}$) of the multilayer material, which is the entire laminate, as shown in Equation (3) below, using the values of the stiffness matrices reset by receiving the thickness information of each layer (k).

[Equation 3]

$$A_{ij} = \sum_{k=1}^{n} Q_{ij}^{k}(z_k - z_{k-1}) \quad B_{ij} = \frac{1}{2}\sum_{k=1}^{n} Q_{ij}^{k}(z_k{}^2 - z_{k-1}{}^2)$$

$$D_{ij} = \frac{1}{3}\sum_{k=1}^{n} Q_{ij}^{k}(z_k{}^3 - z_{k-1}{}^3)$$

In Equation 3, k represents each layer, and the multilayer material is formed of a total of n layers. For example, n is an integer of 2 to 10.

In an exemplary embodiment, in the sixth step, compliance matrices ($[a]_{x,y}$, $[b]_{x,y}$, $[c]_{x,y}$, $[d]_{x,y}$) for the calculated stiffness matrices ($[A]_{x,y}$, $[B]_{x,y}$, $[D]_{x,y}$) of the multilayer material, which is the entire laminate, can be set as shown in Equation 4 below.

[Equation 4]

$$
\begin{bmatrix}
A_{xx} & A_{xy} & A_{xs} & B_{xx} & B_{xy} & B_{xs} \\
A_{yx} & A_{yy} & A_{ys} & B_{yx} & B_{yy} & B_{ys} \\
A_{sx} & A_{sy} & A_{ss} & B_{sx} & B_{sy} & B_{ss} \\
B_{xx} & B_{xy} & B_{xs} & D_{xx} & D_{xy} & D_{xs} \\
B_{yx} & B_{yy} & B_{ys} & D_{yx} & D_{yy} & D_{ys} \\
B_{sx} & B_{sy} & B_{ss} & D_{sx} & D_{sy} & D_{ss}
\end{bmatrix}^{-1}
=
\begin{bmatrix}
a_{xx} & a_{xy} & a_{xs} & b_{xx} & b_{xy} & b_{xs} \\
a_{yx} & a_{yy} & a_{ys} & b_{yx} & b_{yy} & b_{ys} \\
a_{sx} & a_{sy} & a_{ss} & b_{sx} & b_{sy} & b_{ss} \\
c_{xx} & c_{xy} & c_{xs} & d_{xx} & d_{xy} & d_{xs} \\
c_{yx} & c_{yy} & c_{ys} & d_{yx} & d_{yy} & d_{ys} \\
c_{sx} & c_{sy} & c_{ss} & d_{sx} & d_{sy} & d_{ss}
\end{bmatrix}
$$

In an exemplary embodiment, in the seventh step, it is possible to calculate elastic moduli ($/E_{x,y}$), Poisson's ratios ($/\upsilon_{x,y}$), and shear moduli ($/G_{x,y}$) of the multilayer material, which is the entire laminate, using the total thickness (h) and values of the set compliance matrices ($[a]_{x,y}$, $[b]_{x,y}$, $[c]_{x,y}$, $[d]_{x,y}$) of the multilayer material, which is the entire laminate, as shown in Equation 5 below.

[Equation 5]

$$/E_x = \frac{1}{ha_{xx}} \quad /E_y = \frac{1}{ha_{yy}} \quad /G_{xy} = \frac{1}{ha_{ss}}$$

$$/V_{xy} = -\frac{a_{yx}}{a_{xx}} \quad /V_{yx} = -\frac{a_{xy}}{a_{yy}}$$

In one embodiment, the method of the present invention further includes, in the case in which the elastic modulus is selected from the elastic modulus or the thickness of the undetermined layer and input, when the calculated elastic modulus, Poisson's ratio, and shear modulus do not converge to the elastic modulus, Poisson's ratio, and shear modulus received as the final target physical properties, updating the value of the elastic modulus of the undetermined layer.

In another embodiment, the method of the present invention further includes, in the case in which the thickness is selected from the elastic modulus and the thickness of the undetermined layer and input, when the calculated elastic modulus, Poisson's ratio, and shear modulus do not converge to the elastic modulus, Poisson's ratio, and shear modulus received as the final target physical properties, updating the value of the thickness of the undetermined layer.

In still another embodiment, the method of the present invention further includes, when the calculated elastic modulus, Poisson's ratio, and shear modulus do not converge to the elastic modulus, Poisson's ratio and shear modulus received as the final target physical properties, outputs an elastic modulus, Poisson's ratio, shear modulus and thickness of an undetermined layer.

In yet another embodiment, the method of the present invention further includes plotting the correlation between elastic modulus and thickness of the undetermined layer in a graph.

Hereinafter, to describe the present invention in detail so as to be easily implemented by those of ordinary skill in the art to which the present invention belongs, exemplary embodiments of the present invention will be described with reference to the accompanying drawings. Other objects, features, and operational advantages, including the object, action and effect of the present invention will be more apparent by the description of the exemplary embodiments.

For reference, embodiments disclosed herein are merely presented by selecting the most exemplary embodiments to help the understanding of those of ordinary skill in the art among various possible embodiments, and the technical spirit of the present invention is not necessarily limited only by the presented embodiments. Various changes, additions, and modifications including equivalents or substitutes are possible without departing from the technical spirit of the present invention.

In addition, terms or words used in in the specification and claims should not be construed as being limited to general or dictionary meanings, and should not be interpreted with the meaning and concept in accordance with the technical idea of the present invention based on the principle that the inventors have appropriately defined the concepts of the terms in order to explain the present invention in the best way. In one example, singular expressions include plural expressions unless clearly indicated otherwise in the context, expressions regarding the direction are set based on the position expressed on the drawings for convenience of description, and the expression "connect" or "access" includes not only direct connection or access, but also connection or access through a different component between two components. In addition, the expression "unit" includes a unit implemented using hardware, a unit implemented using software, a unit implemented using both hardware and software, and one unit may be implemented using one or more pieces of hardware or software, and two or more units may be implemented using one piece of hardware or software.

**First embodiment**

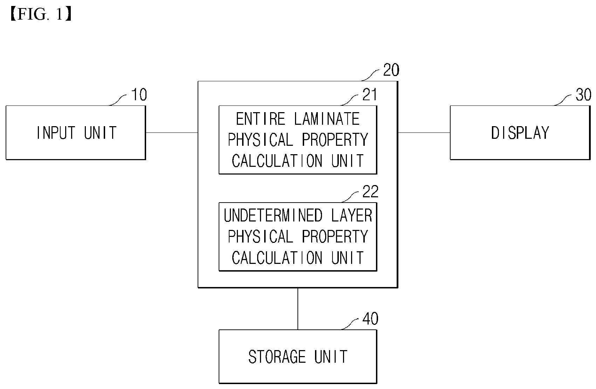

FIG. 1 is a diagram illustrating a configuration of a system for predicting the physical properties of a multilayer material according to a first embodiment of the present invention.

As shown in FIG. 1, the configuration of the system for predicting the physical properties of a multilayer material according to the first embodiment of the present invention is composed of an input unit 10 for inputting, by a user, elastic moduli ($E^k_{1,2}$) in the machine direction (1) and transverse direction (2) of each layer (k), Poisson's ratios ($\upsilon^k_{1,2}$) in the machine direction (1) and transverse direction (2) of each layer (k), shear moduli ($G^K_{1,2}$) in the machine direction (1) and transverse direction (2) of each layer (k), a thickness ($Z^k$) and a set number of zones (N) of each layer (k), except for an undetermined layer, and elastic moduli ($E_{1,2}$) or a thickness (z) of the undetermined layer, a control unit 20 which is connected to the input unit 10 and includes an entire laminate physical property calculation unit 21 and an undetermined layer physical property calculation unit 22, a display 30 connected to the control unit 20, and a storage unit 40 connected to the control unit 20.

In the present invention, the multilayer material refers to a laminate having a structure in which two or more materials are laminated, such as a multilayer film, for example, a polymer. The multilayer material may refer to a composite material of different materials such as a fiber reinforced plastic (FRP) and an aluminum pouch. For example, the multilayer material may refer to a multilayer film.

Meanwhile, the input unit 10 is for inputting actual physical property values by a user, but the present invention is not necessarily limited thereto. For example, the input unit 10 may access a database (DB) in which information to be input to the input unit 10 is stored.

Moreover, in the Poisson's ratios ($\upsilon^k_{1,2}$) in the machine direction (1) and transverse direction (2) of each layer (k), $\upsilon_{1,2}$ refers to a Poisson's ratio associated with transformation in the transverse direction (2) when a force is loaded on the material in a machine direction (1), and $\upsilon_{2,1}$ refers to the Poisson's ratio associated with transformation in the machine direction (1) when the material is loaded in the transverse direction (2). Meanwhile, when the material is an isotropic material, it is possible that $\upsilon_{1,2}=\upsilon_{2,1}=\upsilon$.

Meanwhile, the angle ($\theta^k$) may refer to an angle in the counterclockwise direction of each layer with respect to the x-direction of the multilayer material.

In addition, the thickness ($Z^k$) of each layer (k) may refer to the information on coordinates from the thickness center of the entire laminate in the multilayer material.

The display 30 may provide an input screen in which a name input field, a thickness input field, and an angle input field are arranged for each layer, and a machine direction (MD) elastic modulus input field, a transverse direction (TD) elastic modulus input field, a Poisson's ratio input field, a thermal expansion coefficient input field, a sample size (length, width) input field, an X-axis tensile strength input field, a Y-axis tensile strength input field, a shear strength input field, and a temperature change input field are arranged for each layer.

The display 30 may provide an output screen in which x-axis elastic modulus, y-axis elastic modulus, Poisson's ratio, shear modulus, a volumetric modulus, and a coefficient of thermal expansion are arranged.

**Second embodiment**

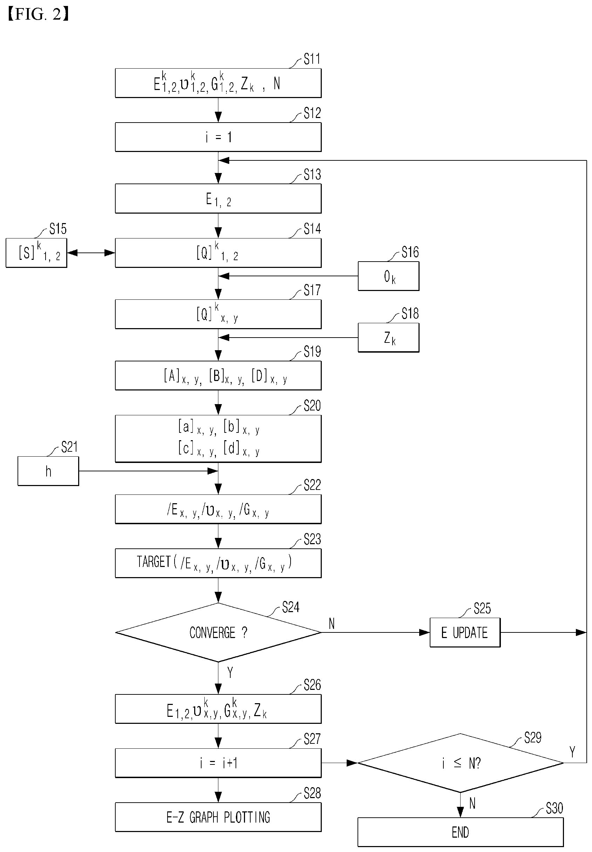

FIG. 2 is a flowchart of a method of predicting the physical properties of a multilayer material according to a second embodiment of the present invention.

As shown in FIG. 2, the method of predicting the physical properties of a multilayer material in which two or more materials are laminated according to a second embodiment of the present invention includes

inputting elastic moduli ($E^k_{1,2}$) in the machine direction (1) and transverse direction (2) of each layer (k), Poisson's ratios ($\upsilon^k_{1,2}$) in the machine direction (1) and transverse direction (2) of each layer (k), shear moduli ($G^k_{1,2}$) in the machine direction (1) and transverse direction (2) of each layer (k), a thickness ($Z^k$) of each layer (k), and a set number of zones (N), except for an undetermined layer (S11),

setting a variable (i) of the number of zones to 1 (S12),

inputting elastic moduli ($E_{1,2}$) of the undetermined layer (S13), and

calculating stiffness matrices ($[Q]^k_{1,2}$) in the machine direction (1) and transverse direction (2) of each layer (k) using the elastic moduli ($E^k_{1,2}$), Poisson's ratios ($\upsilon^k_{1,2}$), and shear moduli ($G^k_{1,2}$) (S14).

[Equation 1]

$$\begin{bmatrix} \sigma_1 \\ \sigma_2 \\ \tau_6 \end{bmatrix} = \begin{bmatrix} Q_{11} & Q_{12} & 0 \\ Q_{12} & Q_{22} & 0 \\ 0 & 0 & Q_{66} \end{bmatrix} \begin{bmatrix} \varepsilon_1 \\ \varepsilon_2 \\ \gamma_6 \end{bmatrix}$$

$$Q_{11} = \frac{E_1}{1 - V_{12} V_{21}} \qquad Q_{22} = \frac{E_2}{1 - V_{12} V_{21}}$$

$$Q_{12} = Q_{21} = \frac{V_{21} E_1}{1 - V_{12} V_{21}} = \frac{V_{12} E_2}{1 - V_{12} V_{21}}$$

$$Q_{66} = G_{12}$$

In Equation 1, for an isotropic material, $G = E / 2(1+\upsilon)$.

The method of predicting the physical properties of a multilayer material includes setting compliance matrices ($[S]^k_{1,2}$) for the calculated stiffness matrices ($[Q]^k_{1,2}$) in the machine direction (1) and transverse direction (2) of each layer (k) (S 15),

inputting a lamination angle ($\theta^k$) in the machine direction (1) of each layer (S16), and

resetting stiffness matrices ($[Q]^k_{x,y}$) of each layer (k) by reflecting the lamination angle ($\theta^k$) of each layer (k) in the obtained stiffness matrices ($[Q]^k_{1,2}$) as shown in Equation 2 below (S 17).

[Equation 2]

$$[T] = \begin{bmatrix} m^2 & n^2 & 2mn \\ n^2 & m^2 & -2mn \\ -mn & mn & m^2 - n^2 \end{bmatrix} \quad m=\cos\theta, \ n=\sin\theta$$

$$\begin{bmatrix} Q_{xx} & Q_{xy} & 2Q_{xs} \\ Q_{xy} & Q_{yy} & 2Q_{ys} \\ Q_{xs} & Q_{ys} & 2Q_{ss} \end{bmatrix} = [T^{-1}] \begin{bmatrix} Q_{11} & Q_{12} & 0 \\ Q_{12} & Q_{22} & 0 \\ 0 & 0 & 2Q_{66} \end{bmatrix} [T]$$

$$\begin{bmatrix} \sigma_x \\ \sigma_y \\ \tau_s \end{bmatrix} = \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix} \begin{bmatrix} \varepsilon_x \\ \varepsilon_y \\ \gamma_s \end{bmatrix} \quad \begin{array}{l} \text{Stress-strain relation reflecting angle} \\ (\text{dir-x,y}) \end{array}$$

In addition, inputting a thickness ($Z^k$) of each layer (k) (S 18) and calculating the stiffness matrices ($[A]_{x,y}$, $[B]_{x,y}$, $[D]_{x,y}$) of the multilayer material, which is the entire laminate, using the values of the stiffness matrices reset by receiving the thickness information of each layer (k) as shown in Equation 3 (S19) are performed.

[Equation 3]

$$A_{ij} = \sum_{k=1}^{n} Q_{ij}^{k} (z_k - z_{k-1}) \quad B_{ij} = \frac{1}{2} \sum_{k=1}^{n} Q_{ij}^{k} (z_k{}^2 - z_{k-1}{}^2)$$

$$D_{ij} = \frac{1}{3} \sum_{k=1}^{n} Q_{ij}^{k} (z_k{}^3 - z_{k-1}{}^3)$$

In Equation 3, k represents each layer, and the multilayer material is formed of a total of n layers. For example, n is an integer of 2 to 10.

Compliance matrices ($[a]_{x,y}$, $[b]_{x,y}$, $[c]_{x,y}$, $[d]_{x,y}$) for the calculated stiffness matrices ($[A]_{x,y}$, $[B]_{x,y}$, $[D]_{x,y}$) of the multilayer material, which is the entire laminate, are set as shown in Equation 4 below (S20).

[Equation 4]

$$\begin{bmatrix} A_{xx} & A_{xy} & A_{xs} & B_{xx} & B_{xy} & B_{xs} \\ A_{yx} & A_{yy} & A_{ys} & B_{yx} & B_{yy} & B_{ys} \\ A_{sx} & A_{sy} & A_{ss} & B_{sx} & B_{sy} & B_{ss} \\ B_{xx} & B_{xy} & B_{xs} & D_{xx} & D_{xy} & D_{xs} \\ B_{yx} & B_{yy} & B_{ys} & D_{yx} & D_{yy} & D_{ys} \\ B_{sx} & B_{sy} & B_{ss} & D_{sx} & D_{sy} & D_{ss} \end{bmatrix}^{-1} = \begin{bmatrix} a_{xx} & a_{xy} & a_{xs} & b_{xx} & b_{xy} & b_{xs} \\ a_{yx} & a_{yy} & a_{ys} & b_{yx} & b_{yy} & b_{ys} \\ a_{sx} & a_{sy} & a_{ss} & b_{sx} & b_{sy} & b_{ss} \\ c_{xx} & c_{xy} & c_{xs} & d_{xx} & d_{xy} & d_{xs} \\ c_{yx} & c_{yy} & c_{ys} & d_{yx} & d_{yy} & d_{ys} \\ c_{sx} & c_{sy} & c_{ss} & d_{sx} & d_{sy} & d_{ss} \end{bmatrix}$$

A total thickness (h) of the multilayer material, which is the entire laminate, is calculated and input (S21), and elastic moduli ($/E_{x,y}$), Poisson's ratios ($/\upsilon_{x,y}$), and shear moduli ($/G_{x,y}$) of the multilayer material, which is the entire laminate, are calculated, as shown in Equation 5 below, using the total thickness (h) and values of the set compliance matrices ($[a]_{x,y}$, $[b]_{x,y}$, $[c]_{x,y}$, $[d]_{x,y}$) of the multilayer material, which is the entire laminate (S22).

[Equation 5]

$$/E_x = \frac{1}{ha_{xx}} \qquad /E_y = \frac{1}{ha_{yy}} \qquad /G_{xy} = \frac{1}{ha_{ss}}$$

$$/V_{xy} = -\frac{a_{yx}}{a_{xx}} \qquad /V_{yx} = -\frac{a_{xy}}{a_{yy}}$$

Next, inputting the elastic moduli ($/E_{x,y}$), Poisson's ratios ($/\upsilon_{x,y}$), and shear moduli ($/G_{x,y}$) as the final target physical properties (S23),

determining whether the calculated elastic moduli ($/E_{x,y}$), Poisson's ratios ($/\upsilon_{x,y}$), and shear moduli ($/G_{x,y}$) converge to the elastic moduli ($/E_{x,y}$), Poisson's ratios ($/\upsilon_{x,y}$), and shear moduli ($/Gx,y$) received as the final target physical properties (S24),

when the calculated elastic moduli ($/E_{x,y}$), Poisson's ratios ($/\upsilon_{x,y}$), and shear moduli ($/G_{x,y}$) do not converge to the elastic moduli ($/E_{x,y}$), Poisson's ratios ($/\upsilon_{x,y}$), and shear moduli ($/Gx,y$) received as the final target physical properties, updating values of elastic moduli ($E_{1,2}$) of the undetermined layer and then jumping to S13 (S25),

when the calculated elastic moduli ($/E_{x,y}$), Poisson's ratios ($/\upsilon_{x,y}$), and shear moduli ($/G_{x,y}$) converge to the elastic moduli ($/E_{x,y}$), Poisson's ratios ($/\upsilon_{x,y}$), and shear moduli ($/Gx,y$) received as the final target physical properties, outputting physical property values (elastic moduli ($E_{1,2}$), Poisson's ratios ($\upsilon^k_{x,y}$) and shear moduli ($G^k_{x,y}$)) and a thickness ($Z^k$) of the undetermined layer (S26),

increasing a variable (i) of the number of zones by 1 (S27),

plotting the correlation between the elastic moduli ($E_{1,2}$) and thickness (z) of the undetermined layer in a graph (S28),

determining whether the variable (i) of the number of zones is a set number of zones (N) or less, and when it is set number of zones (N) or less, jumping to S13 (S29), and

ending the operation when the variable (i) of the number of zones exceeds the set number of zones (N) (S30) are performed.

In the inputting of elastic moduli ($E^k_{1,2}$), Poisson's ratios ($\upsilon^k_{1,2}$), and shear moduli ($G^k_{1,2}$) of each layer (k) (S11), generally, measurement of the shear moduli ($G^K_{1,2}$) of each layer (k) is very difficult, so the layer is assumed to be an isotropic material, and shear moduli ($G^k_{1,2}$) are calculated from the elastic moduli ($E^k_{1,2}$) and the Poisson's ratios ($\upsilon^k_{1,2}$) by the control unit 20 using the relation between an elastic modulus (E), a shear modulus (G), and a Poisson's ratio ($\upsilon$) of the isotropic material ($G = E / 2(1+\upsilon)$).

## Third embodiment

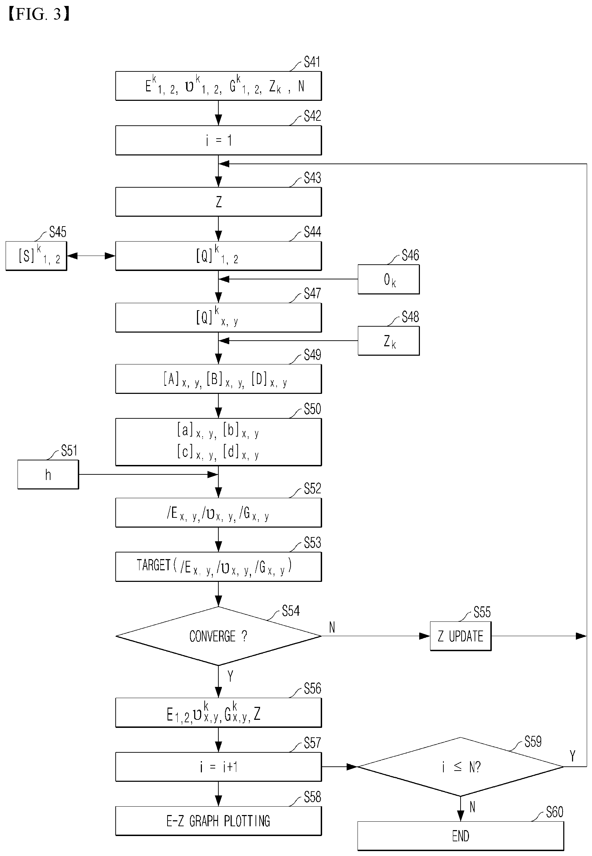

FIG. 3 is a flowchart of a method of predicting the physical properties of a multilayer material according to a third embodiment of the present invention.

As shown in FIG. 3,

the method of predicting the physical properties of a multilayer material in which two or more materials are laminated according to the third embodiment of the present invention includes

inputting elastic moduli ($E^k_{1,2}$) in the machine direction (1) and transverse direction (2) of each layer (k), Poisson's ratios ($\upsilon^k_{1,2}$) in the machine direction (1) and transverse direction (2) of each layer (k), shear moduli ($G^k_{1,2}$) in the machine direction (1) and transverse direction (2) of each layer (k), a thickness ($Z^k$) of each layer (k), and a set number of zones (N), except for an undetermined layer (S41),

setting a variable (i) of the number of zones to 1 (S42),

inputting a thickness (z) of an undetermined layer (S43), and

calculating the stiffness matrices ($[Q]^k_{1,2}$) in the machine direction (a) and transverse matrix (2) of each layer (k) using the elastic moduli ($E^k_{1,2}$), Poisson's ratios ($\upsilon^k_{1,2}$), and shear moduli ($G^k_{1,2}$) as shown in Equation 1 below (S44).

[Equation 1]

$$\begin{bmatrix} \sigma_1 \\ \sigma_2 \\ \tau_6 \end{bmatrix} = \begin{bmatrix} Q_{11} & Q_{12} & 0 \\ Q_{12} & Q_{22} & 0 \\ 0 & 0 & Q_{66} \end{bmatrix} \begin{bmatrix} \varepsilon_1 \\ \varepsilon_2 \\ \gamma_6 \end{bmatrix}$$

$$Q_{11} = \frac{E_1}{1 - v_{12} v_{21}} \qquad Q_{22} = \frac{E_2}{1 - v_{12} v_{21}}$$

$$Q_{12} = Q_{21} = \frac{v_{21} E_1}{1 - v_{12} v_{21}} = \frac{v_{12} E_2}{1 - v_{12} v_{21}}$$

$$Q_{66} = G_{12}$$

In Equation 1, for an isotropic material, G = E / 2(1+υ).

Resetting compliance matrices (inverse matrices) ($[S]^k_{1,2}$) for the calculated stiffness matrices ($[Q]^k_{1,2}$) in the machine direction (a) and transverse matrix (2) of each layer (k) (S45),

inputting a lamination angle ($\theta^k$) of the machine direction (1) of each layer (S46), and
resenting stiffness matrices ($[Q]^k_{x,y}$) of each layer (k) by reflecting the lamination angle ($\theta^k$) of each layer (k) in the obtained stiffness matrices ($[Q]^k_{1,2}$), as shown in Equation 2 below (S47), are performed.

[Equation 2]

$$[T] = \begin{bmatrix} m^2 & n^2 & 2mn \\ n^2 & m^2 & -2mn \\ -mn & mn & m^2 - n^2 \end{bmatrix} \quad m=cos\theta, \ n=sin\theta$$

$$\begin{bmatrix} Q_{xx} & Q_{xy} & 2Q_{xs} \\ Q_{xy} & Q_{yy} & 2Q_{ys} \\ Q_{xs} & Q_{ys} & 2Q_{ss} \end{bmatrix} = [T^{-1}] \begin{bmatrix} Q_{11} & Q_{12} & 0 \\ Q_{12} & Q_{22} & 0 \\ 0 & 0 & 2Q_{66} \end{bmatrix} [T]$$

$$\begin{bmatrix} \sigma_x \\ \sigma_y \\ \tau_s \end{bmatrix} = \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix} \begin{bmatrix} \varepsilon_x \\ \varepsilon_y \\ \gamma_s \end{bmatrix} \quad \begin{array}{l} \text{Stress-strain relation reflecting angle} \\ (dir-x,y) \end{array}$$

Inputting a thickness ($Z^k$) of each layer (k) (S48), and
calculating stiffness matrices ($[A]_{x,y}$, $[B]_{x,y}$, $[D]_{x,y}$) of the multilayer material, which is the entire laminate, using values of the stiffness matrices reset by receiving the thickness information of each layer (k) as shown in Equation 3 below (S49) are performed.

[Equation 3]

$$A_{ij} = \sum_{k=1}^{n} Q_{ij}^{k}(z_k - z_{k-1}) \quad B_{ij} = \frac{1}{2}\sum_{k=1}^{n} Q_{ij}^{k}(z_k^{\wedge}2 - z_{k-1}^{\wedge}2)$$

$$D_{ij} = \frac{1}{3}\sum_{k=1}^{n} Q_{ij}^{k}(z_k^{\wedge}3 - z_{k-1}^{\wedge}3)$$

In Equation 3, k represents each layer, and the multilayer material is formed of a total of n layers. For example, n is an integer of 2 to 10, and specifically, an integer of 3 to 5.

Compliance matrices ($[a]_{x,y}$, $[b]_{x,y}$, $[c]_{x,y}$, $[d]_{x,y}$) for the calculated stiffness matrices ($[A]_{x,y}$, $[B]_{x,y}$, $[D]_{x,y}$) of the multilayer material, which is the entire laminate, are set as shown in Equation 4 below (S50).

[Equation 4]

$$\begin{bmatrix} A_{xx} & A_{xy} & A_{xs} & B_{xx} & B_{xy} & B_{xs} \\ A_{yx} & A_{yy} & A_{ys} & B_{yx} & B_{yy} & B_{ys} \\ A_{sx} & A_{sy} & A_{ss} & B_{sx} & B_{sy} & B_{ss} \\ B_{xx} & B_{xy} & B_{xs} & D_{xx} & D_{xy} & D_{xs} \\ B_{yx} & B_{yy} & B_{ys} & D_{yx} & D_{yy} & D_{ys} \\ B_{sx} & B_{sy} & B_{ss} & D_{sx} & D_{sy} & D_{ss} \end{bmatrix}^{-1} = \begin{bmatrix} a_{xx} & a_{xy} & a_{xs} & b_{xx} & b_{xy} & b_{xs} \\ a_{yx} & a_{yy} & a_{ys} & b_{yx} & b_{yy} & b_{ys} \\ a_{sx} & a_{sy} & a_{ss} & b_{sx} & b_{sy} & b_{ss} \\ c_{xx} & c_{xy} & c_{xs} & d_{xx} & d_{xy} & d_{xs} \\ c_{yx} & c_{yy} & c_{ys} & d_{yx} & d_{yy} & d_{ys} \\ c_{sx} & c_{sy} & c_{ss} & d_{sx} & d_{sy} & d_{ss} \end{bmatrix}$$

In addition, calculating a total thickness (h) of the multilayer material, which is the entire laminate, and inputting the same (S51), and
calculating elastic moduli ($/E_{x,y}$), Poisson's ratios ($/\upsilon_{x,y}$), and shear moduli ($/G_{x,y}$) of the multilayer material, which is the entire laminate, using the total thickness (h) and values of the set compliance matrices ($[a]_{x,y}$, $[b]_{x,y}$, $[c]_{x,y}$, $[d]_{x,y}$) of the multilayer material, which is the entire laminate as shown in Equation 5 below (S52) are performed.

[Equation 5]

$$/E_x = \frac{1}{ha_{xx}} \quad /E_y = \frac{1}{ha_{yy}} \quad /G_{xy} = \frac{1}{ha_{ss}}$$

$$/V_{xy} = -\frac{a_{yx}}{a_{xx}} \quad /V_{yx} = -\frac{a_{xy}}{a_{yy}}$$

Next, inputting the elastic moduli ($/E_{x,y}$), Poisson's ratios ($/\upsilon_{x,y}$) and shear moduli ($/G_{x,y}$) as the final target physical properties (S53),

determining whether the calculated elastic moduli ($/E_{x,y}$), Poisson's ratios ($/\upsilon_{x,y}$), and shear moduli ($/G_{x,y}$) converge to the elastic moduli ($/E_{x,y}$), Poisson's ratios ($/\upsilon_{x,y}$), and shear moduli ($/Gx,y$) received as the final target physical properties (S54),
when the calculated elastic moduli ($/E_{x,y}$), Poisson's ratios ($/\upsilon_{x,y}$), and shear moduli ($/G_{x,y}$) do not converge to the elastic moduli ($/E_{x,y}$), Poisson's ratios ($/\upsilon_{x,y}$), and shear moduli ($/Gx,y$) received as the final target physical properties, updating a value of the thickness (z) of the undetermined layer and then jumping to S13 (S55),
when the calculated elastic moduli ($/E_{x,y}$), Poisson's ratios ($/\upsilon_{x,y}$), and shear moduli ($/G_{x,y}$) converge to the elastic moduli ($/E_{x,y}$), Poisson's ratios ($/\upsilon_{x,y}$), and shear moduli ($/G_{x,y}$) received as the final target physical properties, outputting physical property values (elastic moduli ($E_{1,2}$), Poisson's ratios ($\upsilon^k_{x,y}$), and shear moduli ($G^k_{x,y}$)) and the thickness (z) of the undetermined layer (S56),

increasing a variable (i) of the number of zones by 1 (S57),

plotting the correlation between the elastic moduli ($E_{1,2}$) and thickness (z) of the undetermined layer in a graph (S58),

determining whether the variable (i) of the number of zones is a set number of zones (N) or less, and when it is the set number of zones (N) or less, jumping to S13 (S59), and

terminating the operation when the variable (i) of the number of zones exceeds the set number of zones (N) (S60) are performed.

In the inputting of elastic moduli ($E^k_{1,2}$), Poisson's ratios ($\upsilon^k_{1,2}$), and shear moduli ($G^K_{1,2}$) of each layer (k) (S41), generally, since measurement of the shear moduli ($G^K_{1,2}$) of each layer (k) is very difficult, the layers are assumed to be isotropic materials, and shear moduli ($G^k_{1,2}$) are calculated from the elastic moduli ($E^k_{1,2}$) and the Poisson's ratios ($\upsilon^k_{1,2}$) by the control unit 20 using the relation between an elastic modulus (E), a shear modulus (G) and a Poisson's ratio ($\upsilon$) of the isotropic material (G = E / 2(1+$\upsilon$)).

The actions of the system and method for predicting the physical properties of a multilayer material according to the embodiments of the present invention, which have been configured as above, are as follows.

To predict the physical properties of a multilayer material, a user inputs elastic moduli ($E^k_{1,2}$) in the machine direction (1) and transverse direction (2) of each layer (k, k=1,2,3), Poisson's ratios ($\upsilon^k_{1,2}$) in the machine direction (1) and transverse direction (2) of each layer (k, k=1,2,3), shear moduli ($G^K_{1,2}$) in the machine direction (1) and transverse direction (2) of each layer (k, k=1,2,3), a thickness ($Z^k$) of each layer (k), and a set number of zones (N) while looking at an input screen on a display 30 (S11, S41).

In the input screen provided by the display 30, a name input field, a thickness input field, an angle input field, a machine direction (MD) elastic modulus input field, a transverse direction (TD) elastic modulus input field, a Poisson's ratio input field, a thermal expansion coefficient input field, a sample size (length, width) input field, an X-axis tensile force input field, a Y-axis tensile force input field, a shear force input field, and a temperature change input field are arranged for each layer, and information that is not shown on the input screen is automatically calculated from the information input through the input unit 10 by the control unit 20, and stored in a storage unit 40. For example, when, through the input screens of the input unit 10 and the display 30, a machine direction (MD) elastic modulus ($E^k_1$), a transverse direction (TD) elastic modulus ($E^k_2$), and Poisson's ratios ($\upsilon^k_{1,2}$) are input for each layer, the entire laminate physical property calculation unit 21 in the control unit 20 automatically calculates shear moduli ($G^k_{1,2}$) using the relation between the elastic modulus (E), the shear modulus (G), and the Poisson's ratio ($\upsilon$) of an isotropic material and then stored in the storage unit 40.

$$G = \frac{E}{2(1+\nu)}$$

Next, the undetermined layer physical property calculation unit 22 in the control unit 20 sets the variable (i) of the number of zones to 1 (S12, S42).

Subsequently, to the undetermined layer physical property calculation unit 22 in the control unit 20, elastic moduli ($E_{1,2}$) of the undetermined layer are input (S13), or a thickness (z) of the undetermined layer is input (S43).

Next, the entire laminate physical property calculation unit 21 in the control unit 20 calculates stiffness matrices ($[Q]^k_{1,2}$) in the machine direction (1) and transverse direction (2) of each layer (k) using the elastic moduli ($E^k_{1,2}$), Poisson's ratios ($\upsilon^k_{1,2}$), and shear moduli ($G^k_{1,2}$) as shown in Equation 1 below (S14, S44).

[Equation 1]

$$\begin{bmatrix} \sigma_1 \\ \sigma_2 \\ \tau_6 \end{bmatrix} = \begin{bmatrix} Q_{11} & Q_{12} & 0 \\ Q_{12} & Q_{22} & 0 \\ 0 & 0 & Q_{66} \end{bmatrix} \begin{bmatrix} \varepsilon_1 \\ \varepsilon_2 \\ \gamma_6 \end{bmatrix}$$

$$Q_{11} = \frac{E_1}{1 - V_{12} V_{21}} \qquad Q_{22} = \frac{E_2}{1 - V_{12} V_{21}}$$

$$Q_{12} = Q_{21} = \frac{V_{21} E_1}{1 - V_{12} V_{21}} = \frac{V_{12} E_2}{1 - V_{12} V_{21}}$$

$$Q_{66} = G_{12}$$

In Equation 1, the relation for an isotropic material (G = E / 2(1+υ)) is used.

Subsequently, the entire laminate physical property calculation unit 21 in the control unit 20 calculates inverse matrices ($[S]^k_{1,2}$) for the calculated stiffness matrix($[Q]^k_{1,2}$) in the machine direction (1) and transverse direction (2) of each layer (k) and sets them as compliance matrices (S15, S45). This is for converting a stiffness matrix, which is a singular matrix, into an invertible matrix having an inverse matrix.

Next, to the entire laminate physical property calculation unit 21 in the control unit 20, a lamination angle ($\theta^k$) in the machine direction (1) of each layer is input (S16, S46).

Subsequently, the entire laminate physical property calculation unit 21 in the control unit 20 resets stiffness matrices ($[Q]^k_{x,y}$) of each layer (k) in an x or y direction by reflecting the lamination angle ($\theta^k$) of the multilayer material in the obtained stiffness matrices ($[Q]^k_{1,2}$) of each layer (k) as shown in Equation 2 below (S17, S47).

[Equation 2]

$$[T] = \begin{bmatrix} m^2 & n^2 & 2mn \\ n^2 & m^2 & -2mn \\ -mn & mn & m^2 - n^2 \end{bmatrix} \quad m=\cos\theta,\ n=\sin\theta$$

$$\begin{bmatrix} Q_{xx} & Q_{xy} & 2Q_{xs} \\ Q_{xy} & Q_{yy} & 2Q_{ys} \\ Q_{xs} & Q_{ys} & 2Q_{ss} \end{bmatrix} = [T^{-1}] \begin{bmatrix} Q_{11} & Q_{12} & 0 \\ Q_{12} & Q_{22} & 0 \\ 0 & 0 & 2Q_{66} \end{bmatrix} [T]$$

$$\begin{bmatrix} \sigma_x \\ \sigma_y \\ \tau_s \end{bmatrix} = \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix} \begin{bmatrix} \varepsilon_x \\ \varepsilon_y \\ \gamma_s \end{bmatrix} \quad \text{Stress-strain relation reflecting angle}$$
$$(dir\text{-}x,y)$$

Subsequently, to the entire laminate physical property calculation unit 21 in the control unit 20, a thickness ($Z^k$) of each layer (k) is inputted (S18, S48).

Next, the entire laminate physical property calculation unit 21 in the control unit 20 calculates stiffness matrices ($[A]_{x,y}$, $[B]_{x,y}$, $[D]_{x,y}$) of the multilayer material, which is the entire laminate, using values of the stiffness matrices reset by receiving the thickness information of each layer (k) as shown in Equation 3 below (S19, S49).

[Equation 3]

$$A_{ij} = \sum_{k=1}^{n} Q_{ij}^{k}(z_k - z_{k-1}) \quad B_{ij} = \frac{1}{2}\sum_{k=1}^{n} Q_{ij}^{k}(z_k^2 - z_{k-1}^2)$$

$$D_{ij} = \frac{1}{3}\sum_{k=1}^{n} Q_{ij}^{k}(z_k^3 - z_{k-1}^3)$$

In Equation 3, k represents each layer, and the multilayer material is formed of a total of n layers. For example, n is an integer of 2 to 10.

Subsequently, the entire laminate physical property calculation unit 21 in the control unit 20 sets compliance matrices ($[a]_{x,y}$, $[b]_{x,y}$, $[c]_{x,y}$, $[d]_{x,y}$) for the calculated stiffness matrices ($[A]_{x,y}$, $[B]_{x,y}$, $[D]_{x,y}$) of the multilayer material, which is the entire laminate, as shown in Equation 4 below (S20, S40).

[Equation 4]

$$\begin{bmatrix} A_{xx} & A_{xy} & A_{xs} & B_{xx} & B_{xy} & B_{xs} \\ A_{yx} & A_{yy} & A_{ys} & B_{yx} & B_{yy} & B_{ys} \\ A_{sx} & A_{sy} & A_{ss} & B_{sx} & B_{sy} & B_{ss} \\ B_{xx} & B_{xy} & B_{xs} & D_{xx} & D_{xy} & D_{xs} \\ B_{yx} & B_{yy} & B_{ys} & D_{yx} & D_{yy} & D_{ys} \\ B_{sx} & B_{sy} & B_{ss} & D_{sx} & D_{sy} & D_{ss} \end{bmatrix}^{-1} = \begin{bmatrix} a_{xx} & a_{xy} & a_{xs} & b_{xx} & b_{xy} & b_{xs} \\ a_{yx} & a_{yy} & a_{ys} & b_{yx} & b_{yy} & b_{ys} \\ a_{sx} & a_{sy} & a_{ss} & b_{sx} & b_{sy} & b_{ss} \\ c_{xx} & c_{xy} & c_{xs} & d_{xx} & d_{xy} & d_{xs} \\ c_{yx} & c_{yy} & c_{ys} & d_{yx} & d_{yy} & d_{ys} \\ c_{sx} & c_{sy} & c_{ss} & d_{sx} & d_{sy} & d_{ss} \end{bmatrix}$$

Next, to the entire laminate physical property calculation unit 21 in the control unit 20, a total thickness (h) of the multilayer material, which is the entire laminate, is calculated from the thickness information of each layer (k) and inputted (S21, S51).

Subsequently, the entire laminate physical property calculation unit 21 in the control unit 20 calculates elastic moduli ($/E_{x,y}$), shear moduli ($/G_{x,y}$), and Poisson's ratios ($/\upsilon_{x,y}$) of the multilayer material, which is the entire laminate, using the total thickness (h) and values of the set compliance matrices ($[a]_{x,y}$, $[b]_{x,y}$, $[c]_{x,y}$, $[d]_{x,y}$) of the multilayer material, which is the entire laminate, as shown in Equation 5 below (S22, S52).

[Equation 5]

$$/E_x = \frac{1}{ha_{xx}} \quad /E_y = \frac{1}{ha_{yy}} \quad /G_{xy} = \frac{1}{ha_{ss}}$$

$$/V_{xy} = -\frac{a_{yx}}{a_{xx}} \quad /V_{yx} = -\frac{a_{xy}}{a_{yy}}$$

Next, the user inputs elastic moduli ($/E_{x,y}$), Poisson's ratios ($/\upsilon_{x,y}$), and shear moduli ($/G_{x,y}$) as the final target physical properties using the input unit 10 while looking at the input screen of the display 30, and the undetermined layer physical property calculation unit 22 in the control unit 20 reads the same (S23, S53).

Subsequently, the undetermined layer physical property calculation unit 22 in the control unit 20 determines whether the calculated elastic moduli ($/E_{x,y}$), Poisson's ratios ($/\upsilon_{x,y}$), and shear moduli ($/G_{x,y}$) converge to the elastic moduli ($/E_{x,y}$), Poisson's ratios ($/\upsilon_{x,y}$), and shear moduli ($/Gx,y$) received as the final target physical properties (S24, S54).

When the calculated elastic moduli ($/E_{x,y}$), Poisson's ratios ($/\upsilon_{x,y}$), and shear moduli ($/G_{x,y}$) do not converge to the elastic moduli ($/E_{x,y}$), Poisson's ratios ($/\upsilon_{x,y}$), and shear moduli ($/Gx,y$) received as the final target physical properties,

(i) in the case in which an elastic modulus is selected from the elastic modulus and thickness of the undetermined layer and inputted, the undetermined layer physical property calculation unit 22 in the control unit 20 updates values of the elastic moduli ($E_{1,2}$) of an undetermined layer and jumps to S13 (S25), the process of which is repeated, and (ii) in the case in which a thickness is selected from the elastic modulus or thickness of the undetermined layer and inputted, the undetermined layer physical property calculation unit 22 in the control unit 20 updates a value of the thickness (z) of the undetermined layer and jumps to S13, the process of which is repeated.

Unlike this, when the calculated elastic moduli ($/E_{x,y}$), Poisson's ratios ($/\upsilon_{x,y}$), and shear moduli ($/G_{x,y}$) converge to the elastic moduli ($/E_{x,y}$), Poisson's ratios ($/\upsilon_{x,y}$), and shear moduli ($/Gx,y$) received as the final target physical properties, the undetermined layer physical property calculation unit 22 in the control unit 20 outputs physical property values (elastic moduli ($E_{1,2}$), Poisson's ratios ($\upsilon^k_{x,y}$), and shear moduli ($G^k_{x,y}$)) and a thickness ($Z^k$) of an undetermined layer (S26, S56).

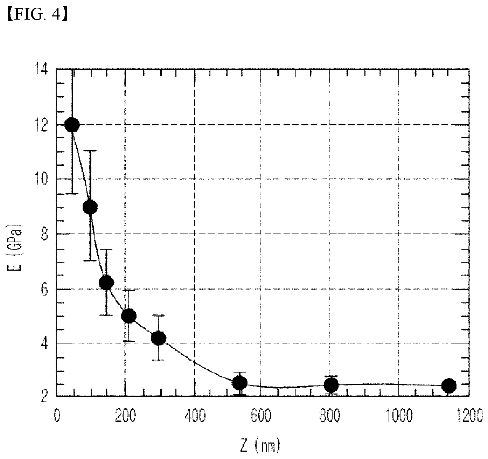

Next, the undetermined layer physical property calculation unit 22 in the control unit 20 increases a variable (i) of the number of zones by 1 (S27, S57), and then plots the correlation between elastic moduli ($E_{1,2}$) and thickness (z) of the undetermined layer in a graph (S28, S58). FIG. 4 is a graph showing the correlation between the elastic moduli ($E_{1,2}$) and the thickness (z) of an undetermined layer according to the present invention.

Subsequently, the undetermined layer physical property calculation unit 22 in the control unit 20 determines whether the variable (i) of the number of zones is the set number of zones (N) or less, and in the case of the variable is the set number of zones (N) or less, jumps to S13 and repeats the above-described process until the variable (i) of the number of zones exceeds the set number of zones (N) (S29, S59).

In the case of the variable (i) of the number of zones is higher than set number of zones (N), the control unit 10 ends the operation (S30, S60).

**Fourth embodiment**

In one embodiment of the present invention, when the values input to the input unit are not immediately obtained, they may be deduced through a conversion process using other physical property values.

In the process of inputting the elastic modulus ($E^k$) of each layer (k) and the Poisson's ratio ($\upsilon^k$) of each layer (k), these values can be calculated using one or more physical property values of a Lame's first parameter ($\lambda^k$), a shear modulus ($G^k$) or a bulk elastic modulus ($K^k$). For example, they can be converted into an elastic modulus ($E^k$) and a Poisson's ratio ($\upsilon^k$) using Formulas 1 to 9 below.

When the available combination is ($\lambda^k$, $G^k$), it is expressed by Formula 1 below.

[Formula 1]

$$E^k = \frac{G^k(3\lambda^k + 2G^k)}{\lambda^k + G^k)} \quad , \qquad \upsilon^k = \frac{\lambda^k}{2(\lambda^k + G^k)}$$

When the available combination is ($\lambda^k$, $E^k$), it is expressed by Formula 2 below.

[Formula 2]

$$\upsilon^k = \frac{A - (E^k + \lambda^k)}{4\lambda^k} \quad , \qquad E^k$$

When the available combination is ($\lambda^k$, $\upsilon^k$), it is expressed by Formula 3 below.

[Formula 3]

$$E^k = \frac{\lambda^k ( 1 + \lambda^k )( 1 - 2\lambda^k )}{\lambda^k} \quad, \quad v^k$$

When the available combination is $(\lambda^k, K^k)$, it is expressed by Formula 4 below.

[Formula 4]

$$E^k = \frac{9K^k ( K^k - \lambda^k )}{3K^k - \lambda^k} \quad, \quad v^k = \frac{\lambda^k}{3K^k - \lambda^k}$$

When the available combination is $(G^k, E^k)$, it is expressed by Formula 5 below.

[Formula 5]

$$v^k = \frac{E - 2G}{2G^k} \quad, \quad E^k$$

When the available combination is $(G^k, \upsilon^k)$, it is expressed by Formula 6 below.

[Formula 6]

$$E^k = 2G^k ( 1 + v^k ) \quad, \quad v^k$$

When the available combination is $(G^k, K^k)$, it is expressed by Formula 7 below.

[Formula 7]

$$E^k = \frac{9K^k G^k}{3K^k + G^k} \quad, \quad v^k = \frac{3K^k - 2G^k}{2( 3K^k + G^k )}$$

When the available combination is $(K^k, E^k)$, it is expressed by Formula 8 below.

[Formula 8]

$$v^k = \frac{3K^k - E^k}{6K^k} \quad, \quad E^k$$

When the available combination is $(K^k, \upsilon^k)$, it is expressed by Formula 9 below.

## [Formula 9]

$$E^k = 3K^k (1 - 2v^k) \quad , \quad v^k$$

The Formulas 1 to 9 above are examples, and it is possible to combine two or more formulas as needed.

[Description of reference numerals]

> 10: Input unit
> 20: Control unit
> 21: Entire laminate physical property calculation unit
> 22: Undetermined layer physical property calculation unit
> 30: Display
> 40: Storage unit

**Claims**

1. A system for predicting physical properties of a multilayer material, comprising:

   > an input unit for inputting (a) any one or more physical properties of an elastic modulus, a Poisson's ratio, a shear modulus, a thickness, a lamination angle, or a set number of zones of each layer, except for an undetermined layer, and (b) any one or more physical properties of an elastic modulus or a thickness of the undetermined layer;
   > a control unit which is connected to the input unit and comprises an entire laminate property calculation unit and an undetermined layer property calculation unit;
   > a display that is connected to the control unit; and
   > a storage unit that is connected to the control unit.

2. The system of claim 1, wherein the control unit is configured to:

   > set compliance matrices for stiffness matrices of the multilayer material, which is an entire laminate, using the input physical properties,
   > calculate the physical properties of the multilayer material, which is the entire laminate, using values of the set compliance matrices, and
   > determine whether the calculated physical properties converge to final target physical properties.

3. The system of claim 1, wherein the input unit is for inputting

   > (a) an elastic modulus, a Poisson's ratio, a shear modulus, a thickness, a lamination angle, and a set number of zones of each layer, except for an undetermined layer, and
   > (b) any one or more of an elastic modulus or thickness of the undetermined layer.

4. The system of claim 1, wherein the control unit, for the multilayer material in which two or more materials are laminated, is configured to:

   > receive (a) the elastic modulus, Poisson's ratio, shear modulus, thickness, lamination angle, and set number of zones of each layer, except for an undetermined layer, and (b) any one or more of the elastic modulus or thickness of the undetermined layer,
   > calculate a stiffness matrix of each layer using the elastic modulus, Poisson's ratio, and shear modulus of each layer, except for the undetermined layer,
   > reset the stiffness matrix of each layer by reflecting the lamination angle of each layer in obtained stiffness matrix, calculates stiffness matrices of the multilayer material, which is the entire laminate, using a value of the stiffness matrix reset by receiving thickness information of each layer, sets compliance matrices for the calculated stiffness matrices of the multilayer material, which is the entire laminate, and calculates any one or more of elastic moduli ($/E_{x,y}$), Poisson's ratios ($/\upsilon_{x,y}$), or shear moduli ($/G_{x,y}$) of the multilayer material, which is the entire laminate, using the total thickness and values of the set compliance matrices of the multilayer material, which is the entire laminate, and

determines whether any one or more of the calculated elastic moduli ($/E_{x,y}$), Poisson's ratios ($/\upsilon_{x,y}$), or shear moduli ($/G_{x,y}$) of the multilayer material converge to any one or more of the target elastic moduli (target $/E_{x,y}$), target Poisson's ratios (target $/\upsilon_{x,y}$), or target shear moduli (target $/G_{x,y}$) received as the final target physical properties.

5. The system of claim 1, wherein,

in the case in which the elastic modulus is selected from the elastic modulus or the thickness of the undetermined layer and input,
when any one or more of the calculated elastic moduli ($/E_{x,y}$), Poisson's ratios ($/\upsilon_{x,y}$), or shear moduli ($/G_{x,y}$) of the multilayer material do not converge to any one or more of the target elastic moduli (target $/E_{x,y}$), target Poisson's ratios (target $/\upsilon_{x,y}$), or target shear moduli (target $/G_{x,y}$) received as final target physical properties, the control unit is configured to update the value of the elastic modulus of the undetermined layer.

6. The system of claim 1, wherein,

in the case in which the thickness is selected from the elastic modulus and the thickness of the undetermined layer and input,
when any one or more of the calculated elastic moduli ($/E_{x,y}$), Poisson's ratios ($/\upsilon_{x,y}$), or shear moduli ($/G_{x,y}$) of the multilayer material do not converge to any one or more of the target elastic moduli (target $/E_{x,y}$), target Poisson's ratios (target $/\upsilon_{x,y}$), or target shear moduli (target $/G_{x,y}$) received as final target physical properties, the control unit updates a value of the thickness of the undetermined layer.

7. The system of claim 1, wherein, when any one or more of the calculated elastic moduli ($/E_{x,y}$), Poisson's ratios ($/\upsilon_{x,y}$), or shear moduli ($/G_{x,y}$) of the multilayer material converge to any one or more of the received target elastic moduli (target $/E_{x,y}$), target Poisson's ratios (target $/\upsilon_{x,y}$), or target shear moduli (target $/G_{x,y}$) as the final target physical properties,
the control unit is configured to output any one or more of the elastic modulus, Poisson's ratio, shear modulus or thickness of the undetermined layer.

8. A method of predicting the physical properties of a multilayer material in which two or more materials are laminated, comprising:

inputting any one or more of physical properties of an elastic modulus, a Poisson's ratio, a shear modulus, a thickness, a lamination angle, or a set number of zones of each layer, except for an undetermined layer, and any one or more physical properties of an elastic modulus or a thickness of the undetermined layer as input values;
calculating physical properties of the multilayer material, which is an entire laminate, using the input physical properties; and
determining whether calculated physical properties converge to final target physical properties.

9. The method of claim 8, wherein the calculating of physical properties of a multilayer material comprises

setting compliance matrices for stiffness matrices of the multilayer material, which is the entire laminate; and
calculating physical properties of the multilayer material, which is the entire laminate, using values of the set compliance matrices.

10. The method of claim 8, wherein the inputting as input values comprises

inputting an elastic modulus, a Poisson's ratio, a shear modulus, a thickness, a lamination angle, and a set number of zones of each layer, except for an undetermined layer (first step); and
inputting any one or more of an elastic modulus or thickness of the undetermined layer (second step).

11. The method of claim 8, wherein the calculating of physical properties of the multilayer material comprises

calculating a stiffness matrix of each layer, except for an undetermined layer, using the elastic modulus, Poisson's ratio, and shear modulus (third step);
resetting a stiffness matrix of each layer by reflecting the lamination angle of each layer in obtained stiffness matrix (fourth step);
calculating stiffness matrices of the multilayer material, which is the entire laminate, using values of the stiffness matrix reset by receiving thickness information of each layer (fifth step);
setting compliance matrices for calculated stiffness matrices of the multilayer material, which is the entire laminate (sixth step); and
calculating elastic moduli, Poisson's ratios, and shear moduli of the multilayer material, which is the entire laminate, using a total thickness and values of the set compliance matrices of the multilayer material, which is the entire laminate (seventh step).

12. The method of claim 8, wherein the determining whether the calculated properties converge to the final target physical properties comprises:

inputting elastic moduli, Poisson's ratios and shear moduli as the final target physical properties (eighth step); and
determining whether calculated elastic moduli ($/E_{x,y}$), Poisson's ratios ($/\upsilon_{x,y}$), and shear moduli ($/G_{x,y}$) converge to target elastic moduli (target $/E_{x,y}$), target Poisson's ratios (target $/\upsilon_{x,y}$), and target shear moduli (target $/G_{x,y}$) input as the final target physical properties (ninth step).

13. The method of claim 11, wherein the calculating of the stiffness matrix of each layer (third step) includes calculating stiffness matrices ($[Q]^k_{1,2}$) in machine direction (1) and transverse direction (2) of each layer (k) using elastic moduli ($E^k_{1,2}$), Poisson's ratios ($\upsilon^k_{1,2}$), and shear moduli ($G^k_{1,2}$) as shown in Equation 1 below:

[Equation 1]

$$\begin{bmatrix} \sigma_1 \\ \sigma_2 \\ \tau_6 \end{bmatrix} = \begin{bmatrix} Q_{11} & Q_{12} & 0 \\ Q_{12} & Q_{22} & 0 \\ 0 & 0 & Q_{66} \end{bmatrix} \begin{bmatrix} \varepsilon_1 \\ \varepsilon_2 \\ \gamma_6 \end{bmatrix}$$

$$Q_{11} = \frac{E_1}{1 - V_{12} V_{21}} \qquad Q_{22} = \frac{E_2}{1 - V_{12} V_{21}}$$

$$Q_{12} = Q_{21} = \frac{V_{21} E_1}{1 - V_{12} V_{21}} = \frac{V_{12} E_2}{1 - V_{12} V_{21}}$$

$$Q_{66} = G_{12}$$

In Equation 1, for an isotropic material, G = E / 2(1+$\upsilon$).

14. The method of claim 11, wherein the resetting of the stiffness matrix of each layer (fourth step) includes resetting stiffness matrices ($[Q]^k_{x,y}$) of each layer (k), as shown in Equation 2 below, by reflecting the lamination angle ($\theta^k$) of each layer (k) in the obtained stiffness matrices ($[Q]^k_{1,2}$):

[Equation 2]

$$[T] = \begin{bmatrix} m^2 & n^2 & 2mn \\ n^2 & m^2 & -2mn \\ -mn & mn & m^2 - n^2 \end{bmatrix} \quad m=\cos\theta, \ n=\sin\theta$$

$$\begin{bmatrix} Q_{xx} & Q_{xy} & 2Q_{xs} \\ Q_{xy} & Q_{yy} & 2Q_{ys} \\ Q_{xs} & Q_{ys} & 2Q_{ss} \end{bmatrix} = [T^{-1}] \begin{bmatrix} Q_{11} & Q_{12} & 0 \\ Q_{12} & Q_{22} & 0 \\ 0 & 0 & 2Q_{66} \end{bmatrix} [T]$$

$$\begin{bmatrix} \sigma_x \\ \sigma_y \\ \tau_s \end{bmatrix} = \begin{bmatrix} Q_{xx} & Q_{xy} & Q_{xs} \\ Q_{xy} & Q_{yy} & Q_{ys} \\ Q_{xs} & Q_{ys} & Q_{ss} \end{bmatrix} \begin{bmatrix} \varepsilon_x \\ \varepsilon_y \\ \gamma_s \end{bmatrix} \quad \begin{array}{l} \text{Stress-strain relation reflecting angle} \\ (\text{dir-x,y}) \end{array}$$

15. The method of claim 11, wherein the calculating of stiffness matrices of the multilayer material (fifth step) includes calculating stiffness matrices ($[A]_{x,y}$, $[B]_{x,y}$, $[D]_{x,y}$) of the multilayer material, which is the entire laminate, as shown in Equation 3 below, using the values of the stiffness matrices reset by receiving the thickness information of each layer (k):

[Equation 3]

$$A_{ij} = \sum_{k=1}^{n} Q_{ij}^{k}(z_k - z_{k-1}) \quad B_{ij} = \frac{1}{2}\sum_{k=1}^{n} Q_{ij}^{k}(z_k{}^2 - z_{k-1}{}^2)$$

$$D_{ij} = \frac{1}{3}\sum_{k=1}^{n} Q_{ij}^{k}(z_k{}^3 - z_{k-1}{}^3)$$

wherein, in Equation 3, k represents each layer, and the multilayer material has a total of n layers.

16. The method of claim 11, wherein the setting of compliance matrices (sixth step) includes setting compliance matrices ($[a]_{x,y}$, $[b]_{x,y}$, $[c]_{x,y}$, $[d]_{x,y}$) for calculated stiffness matrices ($[A]_{x,y}$, $[B]_{x,y}$, $[D]_{x,y}$) of the multilayer material, which is the entire laminate, as shown in Equation 4 below:

[Equation 4]

$$\begin{bmatrix} A_{xx} & A_{xy} & A_{xs} & B_{xx} & B_{xy} & B_{xs} \\ A_{yx} & A_{yy} & A_{ys} & B_{yx} & B_{yy} & B_{ys} \\ A_{sx} & A_{sy} & A_{ss} & B_{sx} & B_{sy} & B_{ss} \\ B_{xx} & B_{xy} & B_{xs} & D_{xx} & D_{xy} & D_{xs} \\ B_{yx} & B_{yy} & B_{ys} & D_{yx} & D_{yy} & D_{ys} \\ B_{sx} & B_{sy} & B_{ss} & D_{sx} & D_{sy} & D_{ss} \end{bmatrix}^{-1} = \begin{bmatrix} a_{xx} & a_{xy} & a_{xs} & b_{xx} & b_{xy} & b_{xs} \\ a_{yx} & a_{yy} & a_{ys} & b_{yx} & b_{yy} & b_{ys} \\ a_{sx} & a_{sy} & a_{ss} & b_{sx} & b_{sy} & b_{ss} \\ c_{xx} & c_{xy} & c_{xs} & d_{xx} & d_{xy} & d_{xs} \\ c_{yx} & c_{yy} & c_{ys} & d_{yx} & d_{yy} & d_{ys} \\ c_{sx} & c_{sy} & c_{ss} & d_{sx} & d_{sy} & d_{ss} \end{bmatrix}$$

17. The method of claim 11, wherein the calculating of elastic moduli, Poisson's ratios, and shear moduli of the multilayer material (seventh step) includes

calculating elastic moduli ($/E_{x,y}$), Poisson's ratios ($/\upsilon_{x,y}$), and shear moduli ($/G_{x,y}$) of the multilayer material, which is the entire laminate, using the total thickness (h) and values of the set compliance matrices ($[a]_{x,y}$, $[b]_{x,y}$, $[c]_{x,y}$, $[d]_{x,y}$) of the multilayer material, which is the entire laminate as shown in Equation 5 below:

[Equation 5]

$$/E_x = \frac{1}{ha_{xx}} \quad /E_y = \frac{1}{ha_{yy}} \quad /G_{xy} = \frac{1}{ha_{ss}}$$

$$/V_{xy} = -\frac{a_{yx}}{a_{xx}} \quad /V_{yx} = -\frac{a_{xy}}{a_{yy}}$$

【FIG. 1】

```
                                        ┌─20
 ┌─10              ┌──────────────────────────┐        ┌─30
 ┌──────────────┐  │         ┌─21              │   ┌──────────────┐
 │              │  │  ┌─────────────────────┐ │   │              │
 │  INPUT UNIT  │──┼──│  ENTIRE LAMINATE    │─┼───│   DISPLAY    │
 │              │  │  │  PHYSICAL PROPERTY   │ │   │              │
 └──────────────┘  │  │  CALCULATION UNIT    │ │   └──────────────┘
                   │  └─────────────────────┘ │
                   │         ┌─22              │
                   │  ┌─────────────────────┐ │
                   │  │  UNDETERMINED LAYER  │ │
                   │  │  PHYSICAL PROPERTY   │ │
                   │  │  CALCULATION UNIT    │ │
                   │  └─────────────────────┘ │
                   └────────────┬─────────────┘
                                │   ┌─40
                        ┌──────────────┐
                        │              │
                        │ STORAGE UNIT │
                        │              │
                        └──────────────┘
```

**【FIG. 2】**

【FIG. 3】

S41 $E^k_{1,2}$, $\upsilon^k_{1,2}$, $G^k_{1,2}$, $Z_k$, N

S42 $i = 1$

S43 Z

S45 $[S]^k_{1,2}$ ⟷ S44 $[Q]^k_{1,2}$

S46 $0_k$

S47 $[Q]^k_{x,y}$

S48 $Z_k$

S49 $[A]_{x,y}$, $[B]_{x,y}$, $[D]_{x,y}$

S50 $[a]_{x,y}$, $[b]_{x,y}$ $[c]_{x,y}$, $[d]_{x,y}$

S51 h

S52 $/E_{x,y}$, $/\upsilon_{x,y}$, $/G_{x,y}$

S53 TARGET ( $/E_{x,y}$, $/\upsilon_{x,y}$, $/G_{x,y}$ )

S54 CONVERGE ? — N → S55 Z UPDATE

Y

S56 $E_{1,2}$, $\upsilon^k_{x,y}$, $G^k_{x,y}$, Z

S57 $i = i+1$ → S59 $i \leq N?$ — Y

N

S60 END

S58 E-Z GRAPH PLOTTING

【FIG. 4】

<table>
<tr><td colspan="2" align="center">**INTERNATIONAL SEARCH REPORT**</td><td>International application No.<br>**PCT/KR2022/012503**</td></tr>
</table>

| **A.** | **CLASSIFICATION OF SUBJECT MATTER** |
|---|---|

**G01N 3/00**(2006.01)i; **G01N 3/06**(2006.01)i; **G01N 3/08**(2006.01)i; **G01N 3/24**(2006.01)i; **G01N 3/60**(2006.01)i; **G01N 33/44**(2006.01)i

According to International Patent Classification (IPC) or to both national classification and IPC

| **B.** | **FIELDS SEARCHED** |
|---|---|

Minimum documentation searched (classification system followed by classification symbols)

G01N 3/00(2006.01); B29C 70/02(2006.01); E04C 3/02(2006.01); G01N 1/28(2006.01); G01N 27/04(2006.01); G01N 3/42(2006.01); G06F 17/10(2006.01); G06F 17/50(2006.01); G06F 19/00(2011.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 다층 소재(multilayer material), 물성(physical property), 예측(predict), 탄성 계수 (modulus of elasticity), 포아송비(Poisson's ratio), 전단 계수(shear modulus), 두께(thickness), 강성 매트릭스(stiffness matrix), 역 행렬(compliance matrix)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | KR 10-1780173 B1 (SABIC GLOBAL TECHNOLOGIES B.V.) 19 September 2017 (2017-09-19)<br>See paragraphs [0025]-[0111], claims 1, 11 and 17 and figures 1-8. | 1,3,5-8,10 |
| A | | 2,4,9,11-17 |
| A | KR 10-2020-0062813 A (UNIST(ULSAN NATIONAL INSTITUTE OF SCIENCE AND TECHNOLOGY)) 04 June 2020 (2020-06-04)<br>See paragraphs [0013]-[0063] and figures 1-5. | 1-17 |
| A | KR 10-2016-0119393 A (INDUSTRY-ACADEMIC COOPERATION FOUNDATION, YONSEI UNIVERSITY) 13 October 2016 (2016-10-13)<br>See claims 1-7 and figure 1. | 1-17 |
| A | KR 10-1200167 B1 (KOREA AEROSPACE INDUSTRIES, LTD.) 13 November 2012 (2012-11-13)<br>See paragraphs [0036]-[0082] and figures 1 and 2. | 1-17 |

☑ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 December 2022** | **05 December 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

EP 4 209 774 A1

## INTERNATIONAL SEARCH REPORT

| International application No. |
| --- |
| **PCT/KR2022/012503** |

**C.    DOCUMENTS CONSIDERED TO BE RELEVANT**

| Category* | Citation of document, with indication, where appropriate, of the relevant passages | Relevant to claim No. |
| --- | --- | --- |
| DA | KR 10-2005-0112788 A (DAEYANG FOUNDATION et al.) 01 December 2005 (2005-12-01) See paragraphs [0020]-[0143], claims 1-4 and figures 2a-7. | 1-17 |

Form PCT/ISA/210 (second sheet) (July 2022)

## INTERNATIONAL SEARCH REPORT
### Information on patent family members

International application No.

**PCT/KR2022/012503**

| Patent document cited in search report | | | Publication date (day/month/year) | Patent family member(s) | | | Publication date (day/month/year) |
|---|---|---|---|---|---|---|---|
| KR | 10-1780173 | B1 | 19 September 2017 | CN | 106796617 | A | 31 May 2017 |
| | | | | CN | 106796617 | B | 25 September 2018 |
| | | | | EP | 3180193 | A1 | 21 June 2017 |
| | | | | JP | 2018-156689 | A | 04 October 2018 |
| | | | | JP | 2018-503884 | A | 08 February 2018 |
| | | | | JP | 6356339 | B2 | 11 July 2018 |
| | | | | US | 2017-0371980 | A1 | 28 December 2017 |
| | | | | WO | 2017-027598 | A1 | 16 February 2017 |
| KR | 10-2020-0062813 | A | 04 June 2020 | KR | 10-2145246 | B1 | 18 August 2020 |
| KR | 10-2016-0119393 | A | 13 October 2016 | KR | 10-1677840 | B1 | 21 November 2016 |
| KR | 10-1200167 | B1 | 13 November 2012 | KR | 10-1102307 | B1 | 05 January 2012 |
| | | | | KR | 10-2010-0119055 | A | 09 November 2010 |
| | | | | KR | 10-2012-0082112 | A | 23 July 2012 |
| KR | 10-2005-0112788 | A | 01 December 2005 | KR | 10-0612032 | B1 | 11 August 2006 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210138914
- KR 1020210138918
- KR 1020220086160
- KR 20050112788